# FASCICULE DE BREVET EUROPEEN

(11) **EP 0 583 178 B1**
(45) Date de publication et mention de la délivrance du brevet: **04.12.1996**
(21) Numéro de dépôt: 93401692.4
(22) Date de dépôt: 30.06.1993
(51) Int. Cl.: C08F 2/32, A61L 15/00

(54) **Perfectionnement pour la préparation de polyacrylates superabsorbants**
Verbesserung in der Herstellung von superabsorbierenden Polyacrylaten
Improvement in the preparation of superabsorbant polyacrylates

(30) Priorité: 12.08.1992 FR 9209960
(43) Date de publication de la demande: 16.02.1994
(73) Titulaire: ELF ATOCHEM S.A., 92800 Puteaux (FR)
(72) Inventeur: Rebre, Shu Rong, F-93400 Vincennes (FR); Collette, Christian, F-75005 Paris (FR); Kowalik, André, F-60270 Gouvieux (FR)
(74) Mandataire: Haicour, Philippe

(56) Documents cités:
- EP-A- 0 441 507

## Description

La présente invention est relative à la production de poudres de polymères acryliques susceptibles d'absorber d'importantes quantités d'eau ou de fluides aqueux.

On sait réaliser des matériaux doués d'une forte capacité d'absorption d'eau par polymérisation en suspension inverse de monomères à insaturation éthylénique, plus particulièrement de monomères acryliques. Les poudres que l'on obtient ainsi gonflent fortement en présence d'eau, en donnant des gels à haute résistance mécanique. Ces propriétés sont mises à profit, entre autres, pour la fabrication d'articles sanitaires pour l'absorption et la rétention de liquides corporels.

Un perfectionnement important dans la production de telles poudres absorbantes est décrit dans le brevet européen EP 0441507. Il consiste à réaliser la polymérisation du monomère acrylique en au moins deux étapes. Dans une première étape, on réalise de façon classique une polymérisation en suspension inverse, polymérisation qui conduit à la formation d'un gel. Dans une seconde étape, on fait absorber à ce gel une nouvelle charge de monomère, et on en provoque la polymérisation au sein même du gel précédemment formé. On peut, le cas échéant, répéter cette séquence d'absorption/polymérisation. On obtient de cette façon des résines polymères d'une granulométrie sensiblement plus forte que les résines de polymérisation en suspension inverse simple. Leur vitesse de gonflement en présence d'eau, le module élastique, la plasticité et la résistance au dégonflement sous pression du gel formé sont également sensiblement améliorés.

Les qualités absorbantes du polymère final dépendent du taux de neutralisation de l'acide acrylique monomère et il est maintenant bien connu que pour l'application hygiène, le taux de neutralisation optimum doit être voisin de 75%, compte tenu en particulier de la compatibilité nécessaire du superabsorbant avec la peau. C'est ainsi que dans le procédé en plusieurs étapes, on provoque une première polymérisation en suspension inverse d'une première charge aqueuse d'acide acrylique neutralisé à 75 %, et qu'on fait absorber par le gel ainsi formé, des charges ultérieures d'acide acrylique aqueux, également neutralisé à 75 %.

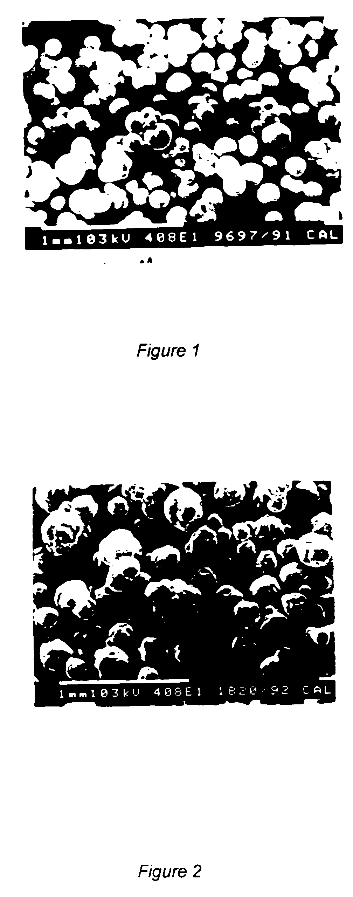

On vient maintenant de trouver qu'il n'était pas indifférent de mettre en oeuvre des solutions de monomère à des taux de neutralisation différents, pourvu que le taux global de neutralisation du polymère final soit respecté. La présente invention est représentée par la revendication 1. En particulier, il est apparu extrêmement avantageux de former un superabsorbant polymère globalement neutralisé à 75 % par le procédé en deux étapes, par la combinaison de deux charges monomères neutralisées, pour la première à un taux compris entre 90 et 100 %, pour la seconde à un taux compris entre 60 et 50 %. Le résultat de cette façon particulière d'opérer est inattendu: d'une part, les particules de polymère obtenues en fin d'opération ne sont plus sphériques (figure 1), mais sphéroïdales bosselées avec des formes qui ne sont pas sans rappeler celles des truffes (figure 2), et d'autre part, leur taille, comprise entre environ 100 et 500µm, est sensiblement plus importante que celle que l'on atteint avec les procédés traditionnels, avec des taux de passant à 100µm très réduits. Il est très appréciable de produire directement les grosses particules qui sont les seules utilisables pour les applications courantes, puisque l'on économise une séquence de post-agglomération. Selon l'art antérieur, on savait faire des particules de cette taille, mais au prix d'une diminution de la charge de monomère : le rendement de l'opération s'en ressent, et les particules que l'on obtient ainsi sont trop polydispersées. Le procédé selon l'invention permet maintenant un accès économique à de grosses particules, de plus à distribution granulométrique resserrée. Il y a également un avantage appréciable à l'utilisation, la géométrie irrégulière des particules en assurant une meilleure fixation dans le milieu fibreux et donc une plus grande facilité de mise en oeuvre.

Les tensioactifs utilisables dans l'invention sont les tensioactifs de l'art antérieur, en particulier les tensioactifs non ioniques tels que les esters d'acides gras et de sorbitan, de polyglycérine, de sucrose, ou les polyoxyéthylène alkylphényl éthers.

Mais il est aussi très avantageux de réaliser la mise en suspension inverse de la première charge de monomère en mettant en oeuvre des tensioactifs de type copolymères-blocs de polyéthylèneglycol/dodécylglycol formés d'une chaîne polyéthylèneglycol munie à l'une et/ou l'autre de leurs extrémités plusieurs chaînes hydrophobes constituées de restes dodécylglycol. On peut alors procéder à l'absorption de la seconde charge de monomère par le gel polymère formé en premier lieu sans qu'il soit besoin de refroidir intensément le réacteur, et opérer cette phase d'absorption à des températures qui ne sont pas inférieures à 35°C, ce qui améliore très sensiblement la rentabilité du procédé.

On peut également utiliser comme tensioactifs de type polymérisable répondant à la formule générale :

R₁-O-(CH₂-CH₂-O)ₙ-R₂

- où R₁ est une chaîne hydrocarbonée à au moins 9 atomes de carbone,
- où R₂ est un groupement polymérisable, reste acryloyle, méthacryloyle ou maléoyle,
- et où le degré de condensation n en oxyde d'éthylène est compris entre 30 et 70. Avec de tels tensioactifs, la phase de d'adsorption de la seconde charge de monomère peut être effectuée à la température de 45°C. Le monoester maléique du nonylphénol oxyéthyléné à 50 molécules d'oxyde d'éthylène est un tensioactif préféré de ce type.

### EXEMPLES

Dans les exemples qui suivent, on distingue les séquences :
a) de préparation de la phase solvant,
b) de préparation de la phase aqueuse de monomère (charge I),
c) de mise en suspension du monomère et polymérisation I,
d) de préparation de la phase aqueuse de monomère (charge II),
e) d'absorption de la charge II et polymérisation II,
f) de séparation du polymère.

### Exemple 1 (art antérieur)

L'exemple correspond à l'obtention en deux étapes d'un polyacrylate neutralisé à 75 % par mise en oeuvre successive de solutions d'acide acrylique toutes deux neutralisées à 75 %.
◆ Séquence a
   Dans un réacteur d'un litre muni d'un dispositif d'introduction de réactifs solides ou liquides, d'un agitateur, d'un système de balayage par gaz neutre, d'une sonde de température et d'un dispositif de chauffage/réfrigération, on dissout à 80°C et sous agitation de 400 tours/minute, 0,92 g de polyéthylène modifié par l'anhydride maléique, un produit commercialisé par Mitsui Petrochemical Industries Co sous le nom de Hi-Wax 1105A et qui servira de colloïde protecteur et 0,736 g de di/tristéarate de saccharose dans 376 g d'heptane. Le mélange est ramené à 38°C.
◆ Séquence b
   A part, on neutralise 92 g d'une solution aqueuse à 80 % en poids d'acide acrylique par 176,8 g de lessive de soude à 17,35 %. On ajoute 0,276 g d'hydroxyéthylcellulose, puis 5,5 g d'une solution aqueuse à 1 % de persulfate de potassium, 0,92 g d'une solution aqueuse à 2 % d'éthylène glycol diglycidyléther. Cette opération est conduite à la température d'environ 15°C.
◆ Séquence c
   Le réacteur étant maintenu sous agitation de 400 tours/minute et sous balayage d'azote à raison de 80 litres/minute, on y introduit peu à peu la phase aqueuse précédemment préparée qui passe en suspension inverse dans l'heptane. La température est portée à 70°C pour provoquer la polymérisation; elle est maintenue à ce niveau pendant 30 minutes. La température est alors ramenée à 20°C.
◆ Séquence d
   Pendant le déroulement de l'opération précédente, on procède à part à la neutralisation de 92 g de solution aqueuse à 80 % en poids d'acide acrylique par 176,8 g de lessive de soude à 17,35 %, puis on ajoute 5,5 g d'une solution aqueuse à 1% de persulfate de potassium et 0,92 g d'une solution aqueuse à 2 % d'éthylène glycol diglycidyléther. On a procédé à la préparation de cette phase aqueuse qui constitue la charge II de monomère à une température d'environ 15°C.
◆ Séquence e
   L'agitation dans le réacteur est portée à 800 tours/minutes, le balayage d'azote étant maintenu à 80 litres/minutes. On y introduit peu à peu la charge II, après quoi la température est portée à 70°C pour engager la seconde phase de polymérisation. On laisse la polymérisation se poursuivre pendant une demi-heure.
◆ Séquence finale f
   On élimine l'heptane et la majeure partie de l'eau par distillation. On ajoute alors au contenu du réacteur 9,2 g d'une solution aqueuse à 2 % d'éthylène glycol diglycidyléther et on poursuit séchage.
   On obtient ainsi une poudre constituée de particules sphériques (figure 1) dont le passant à 100µm atteint 1 %; une microphotographie en est donnée en figure 1.

### Exemple 2

Dans cet exemple, conforme à l'invention, on réalise un polyacrylate avec un taux de neutralisation de 75 %, par mise en oeuvre d'une première solution d'acide acrylique neutralisé à 100 %, puis absorption/polymérisation d'une seconde solution neutralisée à 50 %.

On reproduit l'exemple 1, à la différence près que dans la séquence b, on neutralise les 92 g d'acide polyacrylique à 80 % par 176,8 g de lessive de soude à 23,13 %, et que dans la séquence d, on neutralise les 92 g d'acide polyacrylique à 80 % par 177,7 g de lessive de soude à 11,56 %.

On obtient ainsi une poudre formée de sphéroïdes irréguliers (figure 2) à granulométrie étroite, dont le passant à 100µm est pratiquement nul; une microphotographie en est donnée en figure 2.

### Exemple 3

Dans cet exemple, on combine l'avantage de la combinaison d'une surneutralisation de la première charge de monomère et d'une sous-neutralisation de la seconde charge, avec l'utilisation d'un copolymère bloc polyéthylèneglycol/dodécylglycol, le Dapral E348, commercialisé par AKZO (molécule à environ trois restes dodécylglycol à une extrémité d'une chaîne méthoxypolyéthylène glycol à environ 22 maillons éthylène-glycol), grâce auquel on peut procéder à l'absorption de la seconde charge de monomère par le gel formé au cours de la première polymérisation à la température relativement élevée de 35°C.

Les différences essentielles dans le mode opératoire par rapport à l'exemple 1, sont indiquées ci-après.
◆ Séquence a
   La phase organique est constituée de 285,1 g d'heptane, de 0,92 g de polyéthylène modifié par l'anhydride maléique, un produit commercialisé par Mitsui Petrochemical Industries Co sous le nom de Hi-Wax 1105A et de 0,46 g de Dapral E348.
◆ Séquence b
   La charge I est ici constituée de 92 g de solution aqueuse à 80 % en poids d'acide acrylique, 160,65g de lessive de soude à 24,17 %, 2,75 g d'une solution aqueuse à 2 % de persulfate de potassium et 0,92 g d'une solution aqueuse à 2% d'éthylène glycol diglycidyléther.
◆ Séquence c
   Après polymérisation, la température du contenu du réacteur est ramenée à 36°C
◆ Séquence d
   La charge II est ici constituée de 92 g d'une solution aqueuse à 80 % en poids d'acide acrylique, 135,75 g de lessive de soude à 16,6%, 2,75 g d'une solution aqueuse à 2 % de persulfate de potassium et 0,92 g d'une solution aqueuse à 2% d'éthylèneglycol diglycidyléther, ramenée avant utilisation dans la séquence suivante à la température de 15°C.
   On obtient ainsi une poudre constituée de sphéroïdes à surface irrégulière sans passant notable à 100µm, selon un processus dont la rentabilité est très sensiblement amélioré par à ceux de l'art antérieur qui exigent un refroidissement énergique du réacteur pour aborder la séquence d.

## Revendications

1. Procédé pour la réalisation de poudres superabsorbantes pour l'eau et les liquides aqueux constitués d'acide polyacrylique partiellement neutralisé, procédé comportant les séquences
a) de préparation de la phase solvant,
b) de préparation de la phase aqueuse d'acide acrylique monomère(charge I)
c) de mise en suspension inverse du monomère et polymérisation I,
d) de préparation de la phase aqueuse d'acide acrylique monomère (charge II),
e) d'absorption de la charge II et polymérisation II,
f) de séparation du polymère,
caractérisé en ce que le taux de neutralisation de la charge I est compris entre 90 et 100 % et que celui de la charge II est compris entre 60 et 50 %.

2. Procédé selon la revendication 1, caractérisé en ce que le taux de neutralisation de l'acide polyacrylique final est voisin de 75 %.

3. Procédé selon l'une ou l'autre des revendications 1 à 2, caractérisé en ce que l'acide acrylique monomère de la première charge est mis en suspension inverse à l'aide de tensioactifs polymères-blocs du type copolymère de polyéthyléneglycol/ dodécylglycol.

4. Procédé selon la revendication 3, caractérisé en ce que le polymère bloc est une molécule à environ trois restes dodécylglycol à une extrémité d'une chaîne méthoxypolyéthylène glycol à environ 22 maillons éthylène-glycol.

5. Procédé selon les revendications 3 ou 4, caractérisé en ce que l'absorption de la seconde charge de monomère est réalisée à une température voisine de 35°C.

6. Procédé selon l'une ou l'autre des revendications 1 ou 2, caractérisé en ce que l'acide acrylique monomère de la charge est mis en suspension inverse à l'aide d'un tensioactif polymérisable répondant à la formule générale
R₁-O-(CH₂-CH₂-O)ₙ-R₂
où R₁ est une chaîne hydrocarbonée à au moins 9 atomes de carbone,
où R₂ est un groupement polymérisable, reste acryloyle, méthacryloyle ou maléoyle,
et où le degré de condensation n en oxyde d'éthylène est compris entre 30 et 70.

7. Procédé selon la revendication 6, caractérisé en ce que le tensioactif polymérisable est l'ester maléique de nonylphénol oxyéthyléné à 50 molécules d'oxyde d'éthylène, et qu'il est mis en solution dans la phase solvant organique.

8. Procédé selon les revendications 5 ou 6, caractérisé en ce que l'adsorption de la seconde charge de monomère est réalisée à une température d'environ 45°C.

## Patentansprüche

1. Verfahren zur Herstellung von superabsorbierenden, aus teilweise neutralisierter Polyacrylsäure bestehenden Pulvern für Wasser und wäßrige Flüssigkeiten, wobei dieses Verfahren die folgenden Schritte umfaßt:
a) Herstellung der Lösemittelphase;
b) Herstellung der wäßrigen Phase des Acrylsäuremonomers (Charge I);
c) Inverse Suspendierung des Monomers und Polymerisation I;
d) Herstellung der wäßrigen Acrylsäuremonomer-Phase (Charge II);
e) Absorption der Charge II und Polymerisation II;
f) Abtrennung des Polymers,
dadurch gekennzeichnet, daß der Neutralisationsgrad der Charge I zwischen 90 und 100 % beträgt und daß der der Charge II zwischen 60 und 50 % beträgt.

2. Verfahren nach Anspruch 1, dadurch gekennzeichnet, daß der Neutralisationsgrad der am Ende erhaltenen Polyacrylsäure etwa 75 % beträgt.

3. Verfahren nach Anspruch 1 oder 2, dadurch gekennzeichnet, daß das Acrylsäuremonomer der ersten Charge mit Hilfe von oberflächenaktiven Blockpolymeren des Polyethylenglycol/Dodecylglykol-copolymertyps in inverse Suspension gebracht wird.

4. Verfahren nach Anspruch 3, dadurch gekennzeichnet, daß das Blockpolymer ein Molekül mit etwa 3 Dodecylglykolresten mit einem Ende einer Methoxypolyethylenglykolkette mit etwa 22 Ethylenglykol-Einheiten ist.

5. Verfahren nach Anspruch 3 oder 4, dadurch gekennzeichnet, daß die Absorption der zweiten Charge des Monomers bei einer Temperatur von etwa 35 °C durchgeführt wird.

6. Verfahren nach Anspruch 1 oder 2, dadurch gekennzeichnet, daß das Acrylsäuremonomere der Charge mit Hilfe eines polymerisierbaren oberflächenaktiven Mittels, das der allgemeinen Formel
R₁-O-(CH₂-CH₂-O)ₙ-R₂
entspricht, in inverse Suspension gebracht wird,
wobei R₁ eine Kohlenwasserstoffkette mit mindestens 9 Kohlenstoffatomen ist;
wobei R₂ eine polymerisierbare Gruppe, ein Acryloyl-, Methacryloyl- oder Maleoylrest ist; und
wobei der Kondensationsgrad n an Ethylenoxid zwischen 30 und 70 beträgt.

7. Verfahren nach Anspruch 6, dadurch gekennzeichnet, daß das polymerisierbare oberflächenaktive Mittel der Maleinsäureester des Nonylphenoloxyethylens mit 50 Molekülen Ethylenoxid ist und daß er in der organischen Lösemittelphase in Lösung gebracht wird.

8. Verfahren nach Anspruch 5 oder 6, dadurch gekennzeichnet, daß die Adsorption der zweiten Charge des Monomers bei einer Temperatur von etwa 45 °C durchgeführt wird.

## Claims

1. Process for the production of superabsorbent powders for water and aqueous liquids consisting of partially neutralized polyacrylic acid, process comprising the sequences
a) of preparation of the solvent phase,
b) of preparation of the monomeric acrylic acid aqueous phase (charge I),
c) of placing the monomer in inverse suspension and polymerization I,
d) of preparation of the monomeric acrylic acid aqueous phase (charge II),
e) of absorption of charge II and polymerization II,
f) of isolation of the polymer,
characterized in that the degree of neutralization of charge I is between 90 and 100 % and that that of charge II is between 60 and 50 %.

2. Process according to Claim 1, characterized in that the degree of neutralization of the final polyacrylic acid is close to 75 %.

3. Process according to either of Claims 1 and 2, characterized in that the acrylic acid monomer of the first charge is placed in inverse suspension with the aid of block polymer surfactants of the polyethylene glycol/dodecyl glycol copolymer type.

4. Process according to Claim 3, characterized in that the block polymer is a molecule containing approximately three dodecyl glycol residues with one end of a methoxypolyethylene glycol chain containing approximately 22 ethylene glycol chain units.

5. Process according to Claim 3 or 4, characterized in that the absorption of the second monomer charge is carried out at a temperature close to 35°C.

6. Process according to either of Claims 1 and 2, characterized in that the acrylic acid monomer of the charge is placed in inverse suspension with the aid of a polymerizable surfactant corresponding to the general formula
R₁-O-(CH₂-CH₂-O)ₙ-R₂
where R₁ is a hydrocarbon chain containing at least 9 carbon atoms,
where R₂ is a polymerizable group or acryloyl, methacryloyl or maleoyl residue,
and where the degree of condensation n of ethylene oxide is between 30 and 70.

7. Process according to Claim 6, characterized in that the polymerizable surfactant is the maleic ester of nonylphenol oxyethylenated with 50 molecules of ethylene oxide and in that it is dissolved in the organic solvent phase.

8. Process according to Claim 5 or 6, characterized in that the absorption of the second monomer charge is carried out at a temperature of approximately 45°C.
